# EUROPEAN PATENT APPLICATION

(11) **EP 2 216 041 A1**
(43) Date of publication of application: **11.08.2010**
(21) Application number: 08852946.6
(22) Date of filing: 20.11.2008
(51) Int. Cl.: A61K 38/00, A61P 35/00, A61P 37/04

(54) **METHOD FOR INDUCTION OF CYTOTOXIC T-CELL, CYTOTOXIC T-CELL INDUCER, AND PHARMACEUTICAL COMPOSITION AND VACCINE EACH COMPRISING THE INDUCER**

(30) Priority: 20.11.2007 JP 2007301000
(71) Applicant: NEC Corporation, Tokyo 108-8001 (JP); Kochi University, Kochi-shi Kochi 7808520 (JP)
(72) Inventor: MIYAKAWA, Tomoya, Tokyo 108-8001 (JP); UDAKA, Keiko, Nankoku-shi Kochi 783-8505 (JP)
(74) Representative: Glawe, Delfs, Moll
(86) International application number: PCT/JP2008/003419
(87) International publication number: WO 2009/066462

(57) **Abstract**

A method for inducing cytotoxic T-cells is provided that includes binding to an HLA molecule on the surface of a cell that is a target of a cytotoxic T-cell, a peptide containing one or more types of amino acid sequence selected from the group consisting of SEQ ID NOS: 1, 2, and 3 and composed of not less than 8 and not more than 11 amino acid residues, or a peptide derived from a precursor thereof.

### [Sequence Listing]

SEQUENCE LISTING

<110>  NEC Corporation
       Kochi University

<120>  Method for inducing CTL

<130>  32000051

<160>  9

<170>  PatentIn version 3.1

<210>  1
<211>  9
<212>  PRT
<213>  Homo sapiens

<400>  1

Ala Leu Leu Pro Ala Val Pro Ser Leu
1               5

<210>  2
<211>  9
<212>  PRT
<213>  Homo sapiens

<400>  2

Arg Val Pro Gly Val Ala Pro Thr Leu
1               5

<210>  3
<211>  9
<212>  PRT
<213>  Homo sapiens

<400>  3

Gly Val Phe Arg Gly Ile Gln Asp Val
1               5

<210>  4
<211>  449
<212>  PRT
<213>  Homo sapiens

<400>  4

Met Gly Ser Asp Val Arg Asp Leu Asn Ala Leu Leu Pro Ala Val Pro
1               5                   10                  15

Ser Leu Gly Gly Gly Gly Gly Cys Ala Leu Pro Val Ser Gly Ala Ala
            20                  25                  30

Gln Trp Ala Pro Val Leu Asp Phe Ala Pro Pro Gly Ala Ser Ala Tyr
        35                  40                  45

Gly Ser Leu Gly Gly Pro Ala Pro Pro Pro Ala Pro Pro Pro Pro Pro
    50                  55                  60

Pro Pro Pro Pro His Ser Phe Ile Lys Gln Glu Pro Ser Trp Gly Gly
65                  70                  75                  80

Ala Glu Pro His Glu Glu Gln Cys Leu Ser Ala Phe Thr Val His Phe
                85                  90                  95

Ser Gly Gln Phe Thr Gly Thr Ala Gly Ala Cys Arg Tyr Gly Pro Phe
            100                 105                 110

Gly Pro Pro Pro Pro Ser Gln Ala Ser Ser Gly Gln Ala Arg Met Phe
        115                 120                 125

Pro Asn Ala Pro Tyr Leu Pro Ser Cys Leu Glu Ser Gln Pro Ala Ile
    130                 135                 140

Arg Asn Gln Gly Tyr Ser Thr Val Thr Phe Asp Gly Thr Pro Ser Tyr
145                 150                 155                 160

Gly His Thr Pro Ser His His Ala Ala Gln Phe Pro Asn His Ser Phe
                165                 170                 175

Lys His Glu Asp Pro Met Gly Gln Gln Gly Ser Leu Gly Glu Gln Gln
            180                 185                 190

Tyr Ser Val Pro Pro Pro Val Tyr Gly Cys His Thr Pro Thr Asp Ser
        195                 200                 205

Cys Thr Gly Ser Gln Ala Leu Leu Leu Arg Thr Pro Tyr Ser Ser Asp
    210                 215                 220

Asn Leu Tyr Gln Met Thr Ser Gln Leu Glu Cys Met Thr Trp Asn Gln
225                 230                 235                 240

Met Asn Leu Gly Ala Thr Leu Lys Gly Val Ala Ala Gly Ser Ser Ser
                245                 250                 255

Ser Val Lys Trp Thr Glu Gly Gln Ser Asn His Ser Thr Gly Tyr Glu
            260                 265                 270

Ser Asp Asn His Thr Thr Pro Ile Leu Cys Gly Ala Gln Tyr Arg Ile
        275                 280                 285

His Thr His Gly Val Phe Arg Gly Ile Gln Asp Val Arg Arg Val Pro
    290                 295                 300

Gly Val Ala Pro Thr Leu Val Arg Ser Ala Ser Glu Thr Ser Glu Lys
305                 310                 315                 320

Arg Pro Phe Met Cys Ala Tyr Pro Gly Cys Asn Lys Arg Tyr Phe Lys
                325                 330                 335

Leu Ser His Leu Gln Met His Ser Arg Lys His Thr Gly Glu Lys Pro
            340                 345                 350

Tyr Gln Cys Asp Phe Lys Asp Cys Glu Arg Arg Phe Ser Arg Ser Asp
        355                 360                 365

Gln Leu Lys Arg His Gln Arg Arg His Thr Gly Val Lys Pro Phe Gln
    370                 375                 380

Cys Lys Thr Cys Gln Arg Lys Phe Ser Arg Ser Asp His Leu Lys Thr

385                 390                 395                 400

His Thr Arg Thr His Thr Gly Lys Thr Ser Glu Lys Pro Phe Ser Cys
                405                 410                 415

Arg Trp Pro Ser Cys Gln Lys Lys Phe Ala Arg Ser Asp Glu Leu Val
            420                 425                 430

Arg His His Asn Met His Gln Arg Asn Met Thr Lys Leu Gln Leu Ala
        435                 440                 445

Leu

<210>  5
<211>  9
<212>  PRT
<213>  Homo sapiens

<400>  5

Cys Tyr Thr Trp Asn Gln Met Asn Leu
1               5

<210>  6
<211>  9
<212>  PRT
<213>  Homo sapiens

<400>  6

Thr Tyr Leu Pro Thr Asn Ala Ser Leu
1               5

<210>  7
<211>  9
<212>  PRT
<213>  Homo sapiens

<400>  7

Leu Leu Asp Val Pro Thr Ala Ala Val
1               5

<210>  8
<211>  9
<212>  PRT
<213>  Homo sapiens

<400>  8

Arg Met Phe Pro Asn Ala Pro Tyr Leu
1               5

<210>  9
<211>  9
<212>  PRT
<213>  Homo sapiens

<400>  9

Ser Leu Leu Pro Ala Ile Val Glu Leu
1               5

## Description

### TECHNICAL FIELD

The present invention concerns a method for inducing cytotoxic T-cells, cytotoxic T-cell inducers, and pharmaceutical compositions and vaccines employing them.

### BACKGROUND ART

It is assumed that in one's body malignant cells are spontaneously generated at any time, but they are normally eliminated by natural immunity followed by adaptive immunity, thereby malignant cells are eliminated eventually.

By adaptive immunity, tumor-specific antigens in the body fluid are eliminated by neutralizing antibodies, and the malignant cells are eliminated by cytotoxic T lymphocytes (CTLs). Namely, CTLs recognize specifically tumor-derived antigens (CTL epitopes) composed of 8 to 11 amino acids that are presented by HLA class I molecules on the surface of malignant cells and eliminate those malignant cells by cytolysis. Identifying such tumor-specific CTL epitopes is therefore important to investigate the ongoing specific immunity.

Methods to identify these CTL epitopes and to verify their abilities to induce immune responses are described in Non-Patent Documents 1 to 3.
[Non-Patent Document 1] Y. Oka, O.A. Elisseeva, A. Tsuboi, H. Ogawa, H. Tamaki, H. Li, Y. Oji, E.H. Kim, T. Soma, M. Asada, K. Udaka, E. Maruya, H. Saji, T. Kishimoto, K. Ueda and H. Sugiyama "Human cytotoxic T-lymphocyte response specific for peptides of the wild-type Wilms' tumor gene (WT1) product." Immunogenetics, 51, 99-107, 2000
[Non-Patent Document 2] H. Ohminami, M. Yasukawa and S. Fujita "HLA class I-restricted lysis of leukemia cells by a CD8 + cytotoxic T-lymphocyte clone specific for WT1 peptide." Blood, 95, 286-293, 2000
[Non-Patent Document 3] Gao, L., Bellantuono, I., Elsasser, A., Marley, S.B., Gordon, M.Y., Goldman, J.M. and Stauss, H.J. "Selective elimination of leukemic CD34 + progenitor cells by cytotoxic T lymphocytes specific for WT1." Blood, 95, 2198-2203, 2000
[Non-Patent Document 4] Nakatsuka, S., Oji, Y., Horiuchi, T. et al., "Immunohistochemical detection of WT1 protein in a variety of cancer cells." Modern Pathology 19, 804-814, 2006
[Non-Patent Document 5] Menke, A.L., van der Eb, A.J. and Jochemsen, A.G. "The Wilms' tumor 1 gene: oncogene or tumor suppressor gene?" International Review of Cytology, 181, 151-212, 1998

### DISCLOSURE OF THE INVENTION

The above documents describe HLA-binding peptides which can induce cytotoxic T lymphocytes. However, those peptides alone are not sufficient to treat diseases by tumor antigen-specific immunotherapy.

For example, peptide CMTWNQMNI identified in Non-Patent Documents 1 and 2 (Oka et al., Ohminami et al.) above, is difficult to dissolve in aqueous solution. Moreover, it can form a disulfide-bonded dimer quite easily due to the presence of cysteine in the peptide sequence, thus decreasing the ability to bind to HLA-A*2402 molecule as well as making it further difficult to dissolve. Because of this, it is not easy to work out the effective concentration, and it is also difficult to develop a medicine out of it. In addition, since this CMTWNQMNI peptide has not been found to bind to any other HLA molecule than the HLA-A*2402 molecule, anti-tumor immunotherapy is applicable only to the HLA-A*2402-positive patients. Likewise, peptide RMFPNAPYL, which was identified by non-Patent Document 3 (Gao et al.), has been shown to bind only to the HLA-A*0201 molecule, therefore immunotherapy is applicable only to the HLA-A*0201-positive patients.

Thus, in order to treat or prevent the development of a malignant tumor, it is desirable if more candidate peptides are identified and peptides having advantageous physical properties are selected, and then peptide sequences that can induce cytotoxic T cells are eventually identified.

The present invention has been accomplished over the concern mentioned above, and provides a method to induce cytotoxic T-cells, cytotoxic T-cell inducers, and pharmaceutical compositions and vaccines employing them, that make possible to effectively treat or prevent development of certain tumors.

A method for inducing cytotoxic T-cells according to one exemplary aspect of the invention includes binding to an HLA molecule on the surface of a cell that is a target of a cytotoxic T-cell a peptide comprising one or more types of amino acid sequence selected from the group consisting of SEQ ID NOS: 1, 2, and 3 and composed of not less than 8 and not more than 11 amino acid residues, or a peptide derived from a precursor thereof.

Furthermore, a cytotoxic T-cell inducer according to another exemplary aspect of the invention includes at least one of a peptide and a precursor thereof, the peptide comprising one or more types of amino acid sequence selected from the group consisting of SEQ ID NOS: 1, 2, and 3, composed of not less than 8 and not more than 11 amino acid residues, and binding to an HLA molecule on the surface of a cell that is a target of a cytotoxic T-cell.

Moreover, a pharmaceutical composition for the treatment of a malignant tumor according to another exemplary aspect of the invention includes at least one of a peptide and a precursor thereof, the peptide comprising one or more types of amino acid sequence selected from the group consisting of SEQ ID NOS: 1, 2, and 3, composed of not less than 8 and not more than 11 amino acid residues, and binding to an HLA molecule on the surface of a specific malignant tumor cell.

Furthermore, a vaccine used for the prevention or treatment of a malignant tumor according to another exemplary aspect of the invention includes at least one of a peptide and a precursor thereof, the peptide comprising one or more types of amino acid sequence selected from the group consisting of SEQ ID NOS: 1, 2, and 3, composed of not less than 8 and not more than 11 amino acid residues, and binding to an HLA molecule on the surface of a specific malignant tumor cell.

Out of the present invention, cytotoxic T-cells can be induced effectively, thereby pharmaceutical compositions and vaccines can be developed that are particularly useful for the treatment or prevention of malignant tumors.

### BRIEF DESCRIPTION OF THE DRAWINGS

The above-mentioned object, other objects, features, and advantages will become more obvious by the exemplary embodiments explained below and the attached drawings.

[FIG. 1] A schematic drawing for explaining an active learning experiment design used in an Example.
[FIG. 2] Graphs demonstrating the abilities of peptides to induce cytotoxic T cells elaborated in an Example.
[FIG. 3] Graphs demonstrating the abilities of peptides to induce cytotoxic T cells elaborated in an Example.
[FIG. 4] Graphs demonstrating the abilities of peptides to induce cytotoxic T cells elaborated in an Example..
[FIG. 5] Graphs demonstrating the abilities of peptides to induce cytotoxic T cells elaborated in an Example.
[FIG. 6] Graphs demonstrating the increase of W10-reactive, IFN-γ-producing cells after peptide immunization in patients with malignant tumors who have HLA-A*2402, to be elaborated in an Example.
[FIG. 7] Graphs demonstrating the increase of W10-reactive, IFN-γ-producing cells after peptide immunization in patients with malignant tumors who have HLA-A*0201, to be elaborated in an Example.
[FIG. 8] Graphs demonstrating the increase of W10-reactive, IFN-γ-producing cells after peptide immunization in patients with malignant tumors who have HLA-A*0206, to be elaborated in an Example.
[FIG. 9] Graphs demonstrating cytotoxic activities of peripheral blood mononuclear cells from patients bearing malignant tumors that have been stimulated with HLA-binding peptides. Graphs are elaborated in an Example.
[FIG. 10] Graphs showing changes in diameters of metastatic tumors after starting W10 peptide immunotherapy in adenoid cystic carcinoma patient KB07-006. Graphs are elaborated in an Example.
[FIG. 11] A graph demonstrating the number of IFN-γ-producing cells in peripheral blood mononuclear cells of patient KB07-006 in response to peptides. The graph is elaborated. in an Example.
[FIG. 12] A dotplot showing increase of HLA-24 tetramer-binding CD8 T-cells in peripheral blood mononuclear cells of patient KB07-006 to be elaborated in an Example.
[FIG. 13] A graph showing a tumor suppression effect observed in case KB07-005 after starting W10 peptide immunotherapy.
[FIG. 14] Graphs showing a tumor regulatory activity observed in patient KB07-003 after initiating W10 peptide immunotherapy.

### EXEMPLARY EMBODIMENT

Modes for carrying out the present invention are explained below using the figures.

### <Exemplary Embodiment 1>

The method to induce cytotoxic T-cells in the present exemplary embodiment employs one or more of the peptides selected from a group of peptides composed of SEQ ID NOS: 1, 2, and 3 that bind to the HLA molecules on the surface of target cells and includes precursors thereof of not less than 8 and not more than 11 amino acids in length (hereinafter, called an 'HLA-binding peptide').

All of the amino acid sequences shown in SEQ ID NO: 1 (W10 peptide), SEQ ID NO: 2 (W302 peptide), and SEQ ID NO: 3 (W292 peptide) are sequences composed of 9 amino acid residues present in a particular tumor antigen (Wilms' tumor 1: WT1) shown in SEQ ID NO: 4 which is coded in the genome.

WT1 gene codes for a zinc finger transcription factor and has been first identified as a tumor suppressor factor because its deficiency is related to the development of a childhood kidney malignancy, Wilms' tumor. However, later, it was found that 30 to 80 % of the naturally arising malignant tumors an intact WT1 gene product was overexpressed. Moreover, overexpression of the WT1 product was found to have a positive correlation with the malignant transformation of the cells. Therefore, in mamny malignant tumors it was suggested that the WT1 product acts as an oncogene (Non-Patent Document 5).

Furthermore, these amino acid sequences were identified by a method to predict HLA-binding peptides, which was based on a hypothesis derived using an active learning experiment method (Japanese Patent Application Laid-open No. H11-316754 (1999)), as peptides having 3 or greater in -log Kd values and their HLA-binding abilities have actually been confirmed by conducting HLA-binding assays. These peptides have been further shown to be capable of inducing cytotoxic T-cells.

The reason why an amino acid sequence for which the predicted binding to an HLA molecule in terms of a -log Kd value is 3 or greater is selected as a candidate, is from the viewpoint that in the field of biochemistry it is known that a binding ability, in terms of a -log Kd value, of about 3 can be treated as the threshold level for whether or not a peptide actually binds to an MHC, such as an HLA.

That is, peptides having amino acid sequences shown in SEQ ID NOS: 1, 2, and 3 are considered to be epitope peptides of a WT1 gene product presented in a surface HLA molecule presented on particular malignant tumor cells.

It has been confirmed that peptides having the amino acid sequences shown in SEQ ID NOS: 1, 2, and 3 bind to allelic products of HLA-A genes, i.e., a product of the HLA-A*2402 gene (HLA-A*2402 molecule), a product of the HLA-A*0201 gene (HLA-A*0201 molecule), or a product of the HLA-A*0206 gene

(HLA-A*0206 molecule). About 70 % to 80 % of East Asians including Japanese and close to 50 % of Western Europeans and Americans have one of these allelic types of HLA-A molecule.

The sequences of SEQ ID NOs: 1, 2, and 3 are shown in Table 1, Table 2, and Table 3 below.

**[Table 1]**

| SEQ ID NO: 1 | | | |
|---|---|---|---|
| Amino acid sequence | HLA TYPE | Predicted score | Binding assay data |
| ALLPAVPSL | HLA-A*2402 | 5.6392 | 9.16 |
| ALLPAVPSL | HLA-A*0201 | 5.299 | 7.84 |
| ALLPAVPSL | HLA-A*0206 | 6.2307 | 7.74 |

**[Table 2]**

| SEQ ID NO: 2 | | | |
|---|---|---|---|
| Amino acid sequence | HLA TYPE | Predicted score | Binding assay data |
| RVPGVAPTL | HLA-A*2902 | 5.9548 | 7.67 |
| RVPGVAPTL | HLA-A*0201 | 4.4074 | 6.41 |
| RVPGVAPTL | HLA-A*0206 | 5.9504 | 6.61 |

**[Table 3]**

| SEQ ID NO: 3 | | | |
|---|---|---|---|
| Amino acid sequence | HLA TYPE | Predicted score | Binding assay data |
| GVFRGIQDV | HLA-A*0206 | 5.7901 | 6.48 |

In Table 1, predicted scores and experimentally obtained binding data with respect to binding to the HLA-A*2402 molecule, HLA-A*0201 molecule, and HLA-A*0206 molecule of SEQ ID NO: 1 are shown in terms of -log Kd values.
Furthermore, in Table 2, predicted scores and experimentally obtained binding data with respect to binding to the HLA-A*2402 molecule, HLA-A*0201 molecule, and HLA-A*0206 molecule of SEQ ID NO: 2 are shown in terms of -log Kd values.
Moreover, in Table 3, predicted score and experimentally obtained binding data with respect to binding to the HLA-A*0206 molecule of SEQ ID NO: 3 are shown in terms of -log Kd values.

Such an experiment design method has not been utilized before to develop a method to identify HLA-binding peptides. Therefore, only a very small number of peptides had been known to have HLA-binding abilities by being confirmed experimentally. Because of this, even when a peptide composed of 9 amino acid residues is randomly synthesized by a conventional method and subjected to experiments to bind to an HLA molecule, there was a probability of only about 1 in 100 of finding one that has a binding affinity, in terms of a -log Kd value, exceeding 6.

However, in order to induce cytotoxic T-cells, it is not sufficient to predict the sequences of HLA-binding peptides using the active learning experiment design method but selecting particular amino acid sequences out of those predicted candidates becomes a critical factor.

In particular, peptides of SEQ ID NO: 1 in Table 1 and SEQ ID NO: 2 in Table 2 are peptides having very rare amino acid sequences having high binding abilities to all three different HLA-A allelic molecules. HLA molecules are suggested to have undergone molecular evolution along the course of vertebrate evolution in a way that alleles have been selected so that respective allelic products bind mutually distinct repertoire of peptides. Therefore, peptides that bind to more than one allelic product of HLA molecucle exist rarely (Evolution of the major histocompatibility complex Jan Klein, Felipe Figueroa CRC critical reviews in Immunology volume 6 issue 4, 295-386, 1986). Such peptides that bind to more than one allelic HLA molecules are extremely useful as therapeutic vaccines because they can be used to treat patients with malignant tumors who have one of the allelic HLA molecules by immunotherapy. An active learning experiment design method (Japanese Patent Application Laid-Open Publication No. 11-316754) is an extremely effective method to search for such peptides that bind promiscuously to a number of different allelic HLA molecules.

In the present exemplary embodiment, one or more peptides having amino acid sequence of SEQ ID NOS: 1, 2, and 3 are introduced into a system containing cells that serve as the target cells for cytotoxic T-cells (CTL), bind to HLA class I molecules on the surface of those target cells and presented as antigen peptides to cytotoxic T cells. The cytotoxic T-cells specifically recognize these peptides and are induced to proliferate. Furthermore, the induced cytotoxic T-cells destroy target cells that present the antigen peptides. In this way, the peptides having an amino acid sequence of SEQ ID NOS: 1, 2, and 3 function as tumor antigens (CTL epitopes) to induce cytotoxic T-cells.

The 'inducing' referred to here means generating an activity or an action from a material or a state in which there is almost no activity or action. In particular, 'inducing cytotoxic T-cells' means stimulating the cytotoxic T-cells that specifically recognize a certain antigen into effector cells that have the capability of killing target cells, and/or stimulating the cytotoxic T-cells to proliferate, *in vitro* or *in vivo.*

From another viewpoint, by including one or more types of amino acid sequence selected from the group consisting of SEQ ID NOS: 1, 2, and 3, and precursors thereof, the peptides composed of not less than 8 and not more than 11 amino acid residues, which bind to HLA molecules on the surface of target cells for cytotoxic T-cells, a cytotoxic T-cell inducer can be obtained.

The 'cytotoxic T-cell inducer' means a drug that exhibits an activity to change a state in which CD8 positive T-cells specifically recognizing a certain antigen are not present, or are present only at a very low proportion, into a state in which cytotoxic T-cells recognizing this antigen are present at a very high proportion, and an action of enhancing the capability of individual cytotoxic T-cells of killing a target.

Furthermore, even the HLA-binding peptides containing amino acid sequences that are predicted by the active learning experiment design method and confirmed by the binding experiment to actually, bind, do not always induce cytotoxic T-cells. Therefore, although peptide binding to HLA molecules is a prerequisite, it is not always sufficient to induce cytotoxic T-cells. A difference of T cell repertoire between individuals or some other factors seem to influence the inducibility.

HLA-binding peptides used in the present exemplary embodiment include those that share the sequences cites in SEQ ID NOS: 1, 2, and 3, but have chemical modifications or replacement by different amino acids from the ones coded in their WT1 genomic sequences, such as stereoisomers of amino acids in the positions where substitution do not affect HLA-binding. Furthermore, such HLA-binding peptides may be composed of amino acid residues alone as described above, but they are not particularly limited thereto. For example, it may be an HLA-binding peptide precursor that is optionally modified with a sugar chain or a fatty acid group and the like as long as the activities of the present invention are not impaired. Such a precursor may subject to change by the activity of a digestive enzyme and the like in a living mammalian body such as in a human digestive organ to become an HLA-binding peptide, thus exhibiting similar activities to those shown by the above-mentioned HLA-binding peptides.

In the present exemplary embodiment, cytotoxic T-cells can be induced by peptides that bind to the HLA-A*2402 molecule and HLA-A*0206 molecule, which are prevalent among Asians, such as Japanese, those peptides can be utilized to develop a therapeutic drug, a prophylactic drug, and the like that is particularly effective for the Asians. On the other hand, peptides that bind to the HLA-A*0201 molecule, which is prevalent among Europeans and Americans, can induce cytotoxic T-cells, therefore can be exploited to develop a therapeutic drug, a prophylactic drug, and the like that is particularly effective for the Europeans and Americans.

Moreover, the HLA-binding peptides used in the present exemplary embodiment may be produced using a method known to a person skilled in the art. For example, they may be artificially synthesized by a solid-phase method or a liquid-phase method. Alternatively, these HLA-binding peptides may be produced by expressing them from a DNA fragment or a recombinant vector coding for these HLA-binding peptides. These HLA-binding peptides thus obtained can be identified by a method known to a person skilled in the art. For example, identification is possible by use of Edman degradation, mass spectrometry, and the like.

### <Exemplary Embodiment 2>

The pharmaceutical composition related to the present exemplary embodiment contains the cytotoxic T-cell inducers explained in Exemplary Embodiment 1. That is, this pharmaceutical composition is a pharmaceutical composition for the treatment of malignant tumors, the composition containing at least one of peptides and precursors thereof, the peptides containing one or more types of amino acid sequence selected from the group consisting of SEQ ID NOS: 1, 2, and 3, composed of not less than 8 and not more than 11 amino acid residues, and binding to an HLA molecule on the surface of particular malignant tumor cells.

The peptides having amino acid sequences of SEQ ID NOS: 1, 2, and 3, as explained in Exemplary Embodiment 1, have HLA-binding properties and can induce cytotoxic T-cells.

In the explanation of Exemplary Embodiment 1, cytotoxic T-cells can be induced by using particular malignant tumor cells as targets, and the malignant tumor cells are destroyed by these induced cytotoxic T-cells.

That is, by administering the pharmaceutical composition of the present exemplary embodiment to a patient with a particular malignant tumor, the peptides contained in the composition bind to HLA class I molecules on the surface of cells within the patient's body, and are presented as antigen peptides to cytotoxic T-cells. The cytotoxic T-cells specifically recognize them and are activated. In this way, the cytotoxic T-cells are induced. Furthermore, the induced cytotoxic T-cells destroy the malignant tumor cells presenting the antigen peptides, thus this activity can contribute to the treatment of malignant tumors.

The HLA-binding peptide contained in the present exemplary embodiment may be a peptide consisting of amino acid residues alone as described above, but it is not particularly limited thereto. For example, it may be an HLA-binding peptide precursor that is optionally modified with a sugar chain or a fatty acid group and the like as long as the effects of the present invention are not impaired. Such a precursor may subject to change by the activity of a digestive enzyme and the like in a living mammalian body such as in a human digestive organ to become an HLA-binding peptide, thus exhibiting similar activities to those shown by the above-mentioned HLA-binding peptides. Furthermore, such HLA-binding peptides may be produced by a method known to a person skilled in the art. For example, they may be artificially synthesized by a solid phase method or a liquid phase method.

The pharmaceutical composition of the present exemplary embodiment may be administered to a patient by dissolving it in a water-soluble solvent and made into a preparation in the form of a pharmaceutically acceptable salt.

Examples of the form of such a pharmaceutically acceptable salt include water-soluble salts that are physiologically acceptable, such as sodium, potassium, magnesium, and calcium salts that are buffered at a physiological pH. Other than the water-soluble solvent, a water-insoluble solvent may be used, and examples of such a water-insoluble solvent include alcohols such as ethanol and propylene glycol.

A preparation containing the pharmaceutical composition of the present exemplary embodiment may contain agents for various purposes, and examples of such agents include a preservative and a buffer agent.

Examples of the preservative include sodium bisulfite, sodium bisulfate, sodium thiosulfate, a benzalkonium chloride, chlorobutanol, thimerosal, phenylmercuric acetate, phenylmercuric nitrate, methylparaben, polyvinyl alcohol, phenylethyl alcohol, ammonia, dithiothreitol, and β-mercaptoethanol. Examples of the buffer agent include sodium carbonate, sodium borate, sodium phosphate, sodium acetate, and sodium bicarbonate. These agents can be present in an amount that enables the pH of the system to be maintained at between 2 and 9, and preferably at between 4 and 8.

### <Exemplary Embodiment 3>

The vaccine related to the present exemplary embodiment contains the pharmaceutical composition explained in Exemplary Embodiment 2. That is, this vaccine is a vaccine used for the prevention or treatment of malignant tumors, the vaccine containing at least one of peptides and precursors thereof, the peptide containing one or more types of amino acid sequence selected from the group consisting of SEQ ID NOS: 1, 2, and 3, composed of not less than 8 and not more than 11 amino acid residues, and binding to HLA molecules on the surface of particular cells.

The peptides having an amino acid sequence of SEQ ID NOS: 1, 2, and 3 contained in the pharmaceutical composition, as explained in Exemplary Embodiment 1, have HLA-binding properties and can induce cytotoxic T-cells.

As in Exemplary Embodiment 2, in the explanation of Exemplary Embodiment 1, cytotoxic T-cells can be induced by using particular tumor cells as the targets for the cytotoxic T-cells, and the malignant tumor cells are destroyed by these induced cytotoxic T-cells.

That is, by administering the vaccine of the present exemplary embodiment to a patient with a particular malignant tumor, the peptides contained in the composition bind to HLA molecules on the surface of tissue cells of the patient, including dendritic cells that are resident at an injection site. When cytotoxic T-cells specific for the peptides recognize them, they are activated, proliferate, and circulate via general circulation. When cytotoxic T-cells specific for the peptides enter the tumor tissue, they recognize the same specific tumor antigen peptides naturally bound to HLA molecules present on the surface of tumor cells, and kill those tumor cells. This activity can contribute to the treatment of malignant tumors.

Alternatively, by administering the vaccine of the present exemplary embodiment to a healthy human body, cytotoxic T-cells are induced, the induced cytotoxic T-cells build up within the body, and when particular malignant tumor cells occur, those tumor cells can be destroyed. This activity can contribute to the prevention of malignant tumors.

The HLA-binding peptides contained in the present exemplary embodiment may be peptides consisting of amino acid residues alone as described above, but it is not particularly limited thereto. For example, it may be an HLA-binding peptide precursors that are optionally modified with a sugar chain or a fatty acid group and the like as long as the effects of the present invention are not impaired. Such precursors are subjected to change by the activity of a digestive enzyme and the like in a living mammalian body such as in a human digestive organ to become an HLA-binding peptide, thus exhibiting similar activities to those shown by the above-mentioned HLA-binding peptides. Furthermore, such HLA-binding peptides may be produced by a method known to a person skilled in the art. For example, they may be artificially synthesized by a solid phase method or a liquid phase method.

Furthermore, the vaccines of the present exemplary embodiment may be used in the form of an inactive component-containing vaccine that contains a component other than the pharmaceutical composition, that has no activity itself but has an activity to further enhance the activity of the pharmaceutical composition as vaccines. Examples of the inactive component include an adjuvant and a toxoid.

The pharmaceutical composition of Exemplary Embodiment 2 and the vaccines of Exemplary Embodiment 3 are internally administered by injection or infusion via intradermal, subcutaneous, intravenous, or intramuscular administration, and the like, by per cutaneous administration, or by inhalation via the mucosa of the nose, throat, and the like.
The amount thereof per administration may be set between an amount that can significantly induce cytotoxic T-cells and an amount that does not damage a significant number of non-malingnant cells.

Exemplary embodiments of the present invention are described above, but are exemplifications of the present invention, and various constitutions other than those above may be employed.

### [Examples]

The present invention is further explained below by reference to Examples, but the present invention is not limited thereto.

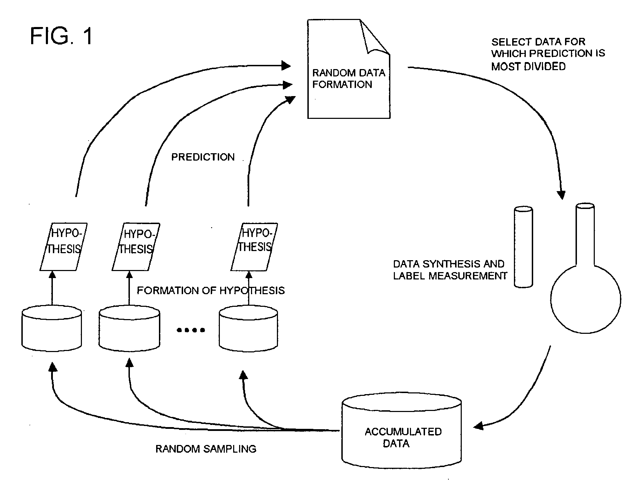

Specifically, procedures of prediction, experiment, and evaluation in the present Examples were carried out based on an active learning experiment design described in WO2006/004182, and in general the following steps were repeated, thus creating rules. A schematic drawing for the active learning experiment design employed here is shown in FIG. 1.

(1) A trial of a lower-order learning algorithm, which will be described later, was carried out once. That is, a number of hypotheses were generated by random sampling from the accumulated data and, with regard to randomly expressed candidate queries (peptides), a query that showed the largest variance of predicted values was selected as a query to be subjected to an experiment.

(2) The peptide of a selected query was synthesized by methods of synthesis and purification, which will be described later, and the actual binding ability was measured by an experiment, which will be described later, and the binding data were added to the accumulated data pool.

According to such an active learning method, the number of peptide-binding assays could be cut down. Otherwise, for peptides composed of 9 amino acid residues, the assay for HLA-binding should have been carried out for all the 500 billion (= 20^9) candidate peptides.

Amino acid sequences shown in SEQ ID NOS: 1, 2, and 3 were extracted by the rules explained above.

### <Synthesis and purification of peptide>

Peptides having the amino acid sequences of SEQ ID NOS: 1, 2, and 3 were manually synthesized by the Merrifield solid-phase method using Fmoc amino acids. After deprotection, reverse phase HPLC purification was carried out using a C18 column to reach a purity of 95 % or higher. Identification of the peptides and confirmation of their purity were carried out using a MALDI-TOF mass spectrometer (Voyager DE RP, PerSeptive). Determination of the peptide concentration was carried out by a Micro BCA assay (Pierce Corp.) using BSA as a standard protein.

### <Experiment of peptide binding to HLA-A*2402 molecule>

The ability of a peptide to bind to an HLA-A*2402 molecule, which is a product of the HLA-A*2402 gene, was measured using C1R-A24 cells expressing the HLA-A*2402 molecule (cells generated by Professor Masafumi Takiguchi, Kumamoto University being supplied with permission by Associate Professor Masaki Yasukawa, Ehime University).

C1R-A24 cells were first exposed to acidic conditions at a pH of 3.3 for 30 seconds, thus dissociating and removing a β2m light chain, which is associated with HLA class I molecules in common, and an endogenous peptide originally bound to the HLA-A*2402 molecule. After neutralization, purified β2m was added to C1R-A24 cells, the obtained product was added to serial dilutions of a peptide, and incubated on ice for 4 hours. Staining was carried out using fluorescently-labeled monoclonal antibody 17A12, which recognizes a complex of the three members (MHC-pep), that is, the HLA-A*2402 molecule, the peptide, and β2m, which had reassociated during the incubation.

Subsequently, the number of MHC-pep complexes per C1R-A24 cell (proportional to the intensity of fluorescence of the above-mentioned fluorescent antibody) was quantitatively measured using a FACScan fluorescence-activated flow cytometer (Becton Dickinson Biosciences). A dissociation constant Kd value between the HLA-A*2402 molecule and the peptide was calculated from the average intensity of fluorescence per cell by a method published in the literature (Udaka et al., Immunogenetics, 51, 816-828, 2000).

### <Experiment of binding peptide to HLA-A*0201 molecule>

The ability of a peptide to bind to an HLA-A*0201 molecule, which is a product of the HLA-A*0201 gene, was measured using strain JY cells expressing the HLA-A*0201 molecule.

JY cells were first exposed to acidic conditions at a pH of 3.8 for 30 seconds, thus dissociating and removing a β2m light chain and an endogenous peptide, which were noncovalently associated with the HLA-A*0201 molecule. After neutralization, a reconstitution experiment was carried out.
The above-mentioned JY cells and the purified β2m were added to serial dilutions of peptide for which the binding ability needs to be measured, and incubation was carried out on ice for 4 hours. HLA-A*0201 molecules that had reassociated up to this time point were stained using the fluorescently-labeled monoclonal antibody BB7.2, a conformation dependent monoclonal Ab specific for an assembled form of HLA-A*0201 molecule.

Subsequently, the amount of fluorescence per cell was measured using a flow cytometer and a dissociation constant Kd value was calculated by a method published in the literature (Udaka et al., Immunogenetics, 51, 816-828, 2000).

### <Experiment of binding peptide to HLA-A*0206 molecule>

The ability of a peptide to bind to an HLA-A*0206 molecule, which is a product of the HLA-A*0206 gene, was measured using RA2.6 cells (cell line newly generated in Kochi University), wherein cDNA of the HLA-A*0206 gene is introduced into RMAS, which is a mouse TAP (transporter associated with antigen processing)-deficient cell line.

RA2. 6 cells were first cultured overnight at 26°C, during this incubation HLA-A*0206 molecules devoid of bound peptide accumulated. Serial dilutions of peptide were added to these cells and peptide binding was carried out at room temperature for 30 minutes.
Subsequently, culture was carried out at 37°C for 3.5 hours, during this incubation empty HLA-A*0206 molecules to which no peptide was bound were denatured, and the tertiary structure was lost.
The cells were stained by adding thereto fluorescently-labeled monoclonal antibody BB7.2, which specifically recognizes the peptide-bound HLA-A*0206 molecule, and incubating on ice for 20 minutes.

Subsequently, the amount of fluorescence per cell was measured using a flow cytometer, and a dissociation constant Kd value was calculated by a method published in the literature (Udaka et al., Immunogenetics, 51, 816-828, 2000).

### <Evaluation results from binding experiment>

As a result, the experimental results were obtained as shown in Table 1, Table 2, and Table 3 above together with the predicted results.

The sequences of SEQ ID NOs: 1, 2, and 3 are derived from a full-length sequence of a WT1 protein consisting of 449 amino acid residues which is registered as M80232 in GENBANK (SEQ ID NO: 4:

MGSDVRDLNALLPAVPSLGGGGGCALPVSGAAQWAPVLDFAPPGASAYGSLGGPAPP
PAPPPPPPPPPHSFIKQEPSWGGAEPHEEQCLSAFTVHFSGQFTGTAGACRYGPFGP
PPPSQASSGQARMFPNAPYLPSCLESQPAIRNQGYSTVTFDGTPSYGHTPSHHAAQF
PNHSFKHEDPMGQQGSLGEQQYSVPPPVYGCHTPTDSCTGSQALLLRTPYSSDNLYQ
MTSQLECMTWNQMNLGATLKGVAAGSSSSVKWTEGQSNHSTGYESDNHTTPILCGAQ
YRIHTHGVFRGIQDVRRVPGVAPTLVRSASETSEKRPFMCAYPGCNKRYFKLSHLQM

HSRKHTGEKPYQCDFKDCERRFSRSDQLKRHQRRHTGVKPFQCKTCQRKFSRSDHLK

THTRTHTGKTSEKPFSCRWPSCQKKFARSDELVRHHNMHQRNMTKLQLAL).

### <Cytotoxic T-cell induction experiment>

The following experiment was carried out using a human blood sample.

### (1) Activation of cytotoxic T-cells

Blood was taken from an HLA-A*2402, HLA-A*0201, or HLA-A*0206-positive patient bearing malignant tumor or a healthy individual from whom informed consent had been obtained in advance.

A method by Azuma et al. (Myeloma cells are highly sensitive to the granule exocytosis pathway mediated by WT1-specific cytotoxic T lymphocytes. Clinical Cancer Research 10, 7402-7412, 2004) was followed. PBMC (Peripheral Blood Mononuclear Cells) were suspended at 5 x 10^5 cells/mL, and stimulated by adding peptide to a concentration of 1 µM.

The culture liquid used was DMEM (Dulbecco's modified minimum essential medium) to which 10 % human serum, 10 U/mL human IL-2, 5 ng/mL human IL-7, and 100 pg/mL human IL-12 were added. 1 U (unit) of IL-2 is the amount of IL-2 necessary for inducing half of the maximum proliferation reaction of IL-2d-dependent cell line CTLL-2 (Gearing, A.J.H. and C.B. Bird, 1987 Lymphokines and interferons, A practical approach, Clemens, M.J. et al., eds. IRL Press. p. 296).

After 1 week, the culture was stimulated with mitomycin C-treated autologous PBMC which had been pulsed with 1 µM of peptide so that it bound to the surface HLA molecules on PBMC. After another week, stimulation was repeated in the same manner.

### (2) ELISPOT assay

On the day after the third in vitro antigen stimulation, 10 U/mL of recombinant human IL-2 was added and incubated for 8-14 days. Cells were subjected to an ELISPOT assay.

Responder cells that had been repeatedly stimulated as described above were harvested and counted, and added at a density of 3.3 x 10^3 to 2 x 10^4 cells per well to a 96-well plate specified for ELISPOT use which had been coated with anti-interferon-γ (IFN-γ) monoclonal antibody. As stimulator cells, C1R-A24 cells were used for HLA-A*2402 molecule-binding WT1 peptide, and JY cells were used for HLA-A*0201 molecule-binding WT1 peptide, and RA2.6 cells were used for HLA-A*0206 molecule-binding WT1 peptide. The stimulator cells had been loaded with peptide by an acid dissociation/reconstitution method (Mashiba et al., Immunogenetics, 59, 197-209 2007) and then treated with mitomycin C before adding to responder cells. After 20 hours, the cells were removed, and anti-IFN-γ antibody was added. An HRPO-labeled secondary antibody was then added and IFN-γ-producing cells were visualized by color development, and counted.

### <Results of Induction Experiment>

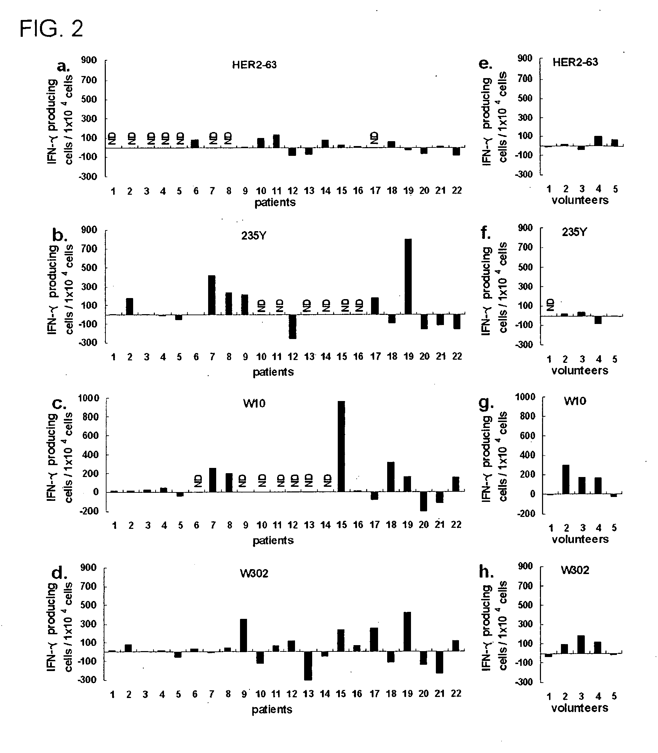

Peripheral blood mononuclear cells (PBMCs) from patients with malignant tumors or healthy volunteers were examined for their genotypes and PBMCs from the HLA-A*2402, HLA-A*0201, or HLA-A*0206-positive individuals were stimulated by one of the peptides once a week for a total of three times in an in vitro culture system. An ELISPOT assay was then carried out, and the results are shown in FIG. 2, FIG. 3, and FIG. 4.

FIG. 2 shows the number of cells secreting interferon-γ (IFN-γ) in a peptide dependent fashion, in PBMCs from the patients with malignant tumors or healthy volunteers having HLA-A*2402. The values shown are the numbers of IFN-γ-producing cells per 10^4 cultured PBMCs, that have been induced *in vitro* by stimulating with HER2-63 (SEQ ID NO: 6), 235Y (SEQ ID NO: 5), W10, or W302 peptide, respectively. Production of IFN-γ was induced by C1R-A24 cells preloaded with the respective peptide. The number of IFN-γ-producing cells in the PBMCs of each person, stimulated *in vitro* without peptide, and induced in response to C1R-A24 having no peptide bound thereto has been subtracted from the numbers obtained against peptide-loaded stimulators. HER2-63 is an HLA-A*2402 binding breast cancer-specific tumor antigen peptide that is not related to WT1 and was used as a control. 235Y is a peptide that is currently used by others in WT1 peptide immunotherapy (A. Tsuboi et al., "Enhanced induction of human WT1-specific cytotoxic T lymphocytes with a 9-mer WT1 peptide modified at HLA-A*2402-binding residues" Cancer Immunol Immunother., 51, 614-620, 2002). The bar graphs show the number of IFN-γ-producing cells of patients (a, b, c, and d in the figure, 22 people each) and healthy volunteers (e, f, g, and h in the figure, 5 people each). There is a tendency for peripheral blood of patients with malignant tumors to have a larger number of cells that react with WT1-derived peptides. ND: not done, this means that ELISPOT assay data could not be obtained.

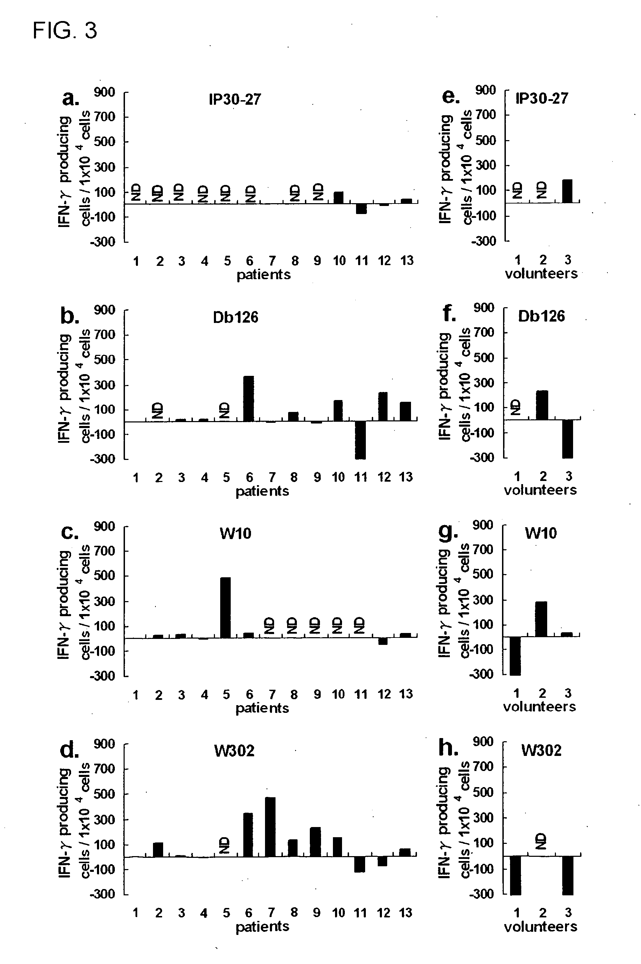

FIG. 3 shows the number of cells secreting interferon-γ (IFN-γ) in a peptide dependent fashion, in PBMCs from the patients with malignant tumors or healthy volunteers having HLA-A*0201. IP30-27 (SEQ ID NO: 7), which is an HLA-A*0201 molecule-binding tumor antigen peptide, but not related to WT1, was used as a negative control. IP30-27 is an HLA-A*0201 molecule-binding peptide identified by Hunt et al., and Db126 (SEQ ID NO: 8) is an HLA-A*0201 molecule-binding WT1 peptide identified by Stauss et al. There is a tendency for peripheral blood of patients with malignant tumors to have a larger number of cells that react with WT1-derived peptides.

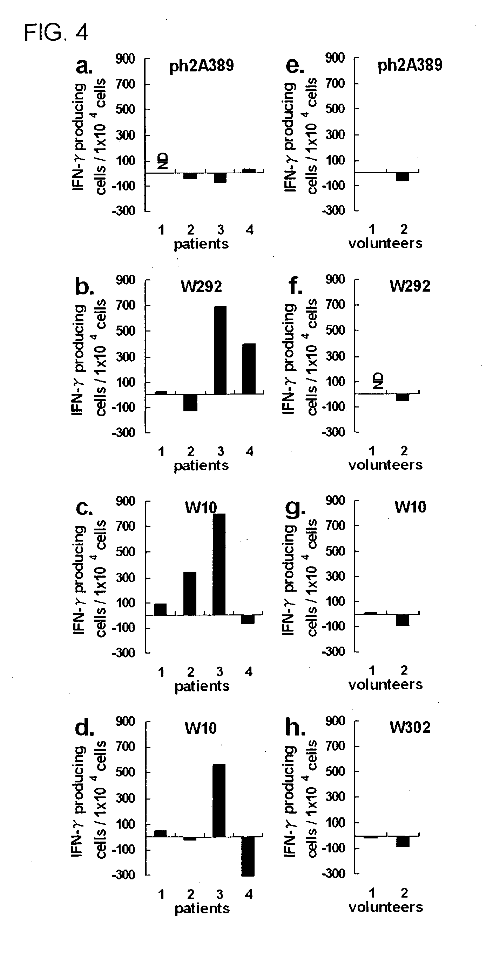

FIG. 4 shows the number of cells secreting interferon-γ (IFN-γ) in a peptide dependent fashion, in PBMCs from the patients with malignant tumors or healthy volunteers having HLA-A*0206. ph2A389 (SEQ ID NO: 9) is a peptide having high binding properties to the HLA-A*0206 molecule and was used as a negative control. There is a tendency for peripheral blood of patients with malignant tumors to have a larger number of cells that react with WT1-derived peptides.

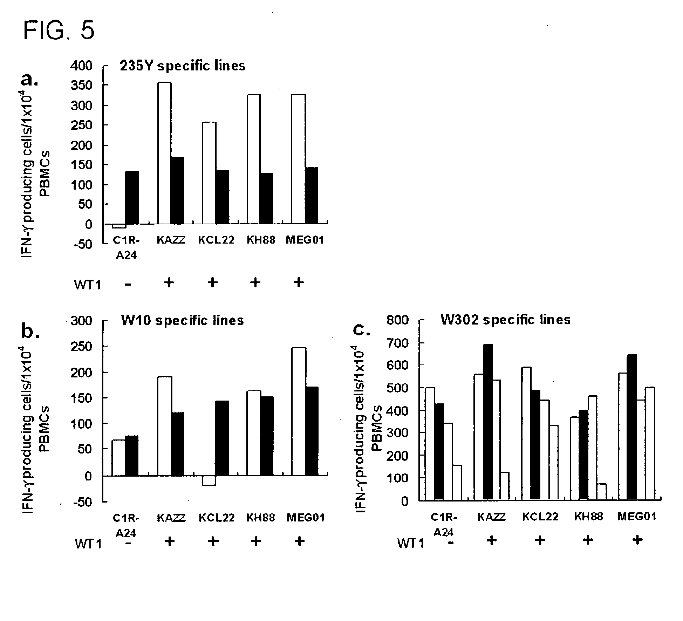

FIG. 5 shows the reactivity of HLA-A*2402 restricted, WT1 peptide-specific cell lines toward tumor cells. It shows the number of IFN-γ-producing cells when 4 different HLA-A*2402 positive cell lines that express WT1 naturally (KAZZ, KCL22, KH88, MEG01) and C1R-A24 cells, which do not express WT1, as a negative control were respectively added as targets to peptide-dependent cell lines (a. 235Y, b. W10, c. W302) established from HLA-A*2402 positive patients. Reactions of cell lines established from different patients are arranged in a bar graph. Compared with a response against C1R-A24 cells, which do not express WT1, reactivity toward the four cell lines expressing WT1 tends to be higher although differences are small.

### <Measurement of cytotoxic T-cell activity after WT1 peptide immunotherapy in patients with malignant tumors >

### 1. Determination of standard immunization protocol

In order to investigate the ability of WT1 peptides to induce cytotoxic T-cells *in vivo,* a phase I/II clinical trial was set up. Patients having any one of HLA-A*0201, HLA-A*0206, and HLA-A*2402 were injected intradermally with W10 peptide on its own or together with a whole-cell pertussis vaccine once a week for a total of three times. In a safety test, the dose was increased stepwise such that 1 mg of W10 peptide was injected to 3 people, and then 3 mg was injected to 6 people. Patients were monitored for adverse events after the peptide administration in accordance with evaluation criteria for an adverse event, CTCAE (Common Terminology Criteria for Adverse Events v3.0 by JCOG/JSCO-Oct. 27, 2004).

No adverse event of Grade 2 or higher was observed during and up to 1 week after the three administrations. Therefore, as a next step, 5 x 10^8 cells worth whole-cell pertussis vaccine, determined by the O.D. 600 value, was admixed to 1 mg of W10 peptide and injected intradermally once a week for a total of three times. Adverse event was monitored up to 1 week after the last immunization. Since there was no adverse event of Grade 2 or higher for 6 people, subsequently, 3 mg of W10 peptide was mixed with the same amount of pertussis whole cell vaccine, and 6 people were immunized. No adverse event of Grade 2 or higher was observed up to 1 week after a total of three injections. Based on these observations, thereafter, an immunization protocol in which 3 mg of W10 peptide was mixed with 5 x 10^8 cells of the whole-cell pertussis for intradermal injection was defined as a standard, thus carrying out the phase I/II trial.

### 2. Measurement of cytotoxic T-cell activity after WT1 peptide immunization

Patients with malignant tumors were injected intradermally once a week for a total of three times by an immunization protocol in which 3 mg of W10 peptide and 5 x 10^8 cells of the whole-cell pertussis vaccine were mixed for injection.

In order to compare the cytotoxicity of peripheral blood mononuclear cells between that before administration of 5 x 10^8 cells of whole-cell pertussis vaccine and 3 mg of W10 peptide to that after administering it three times, peripheral blood mononuclear cells were cultured with the immunizing peptide under conditions described later, and the number of cells that produced IFN-γ in a peptide-dependent manner were counted. The cells used for the ELISPOT assay are also described later.

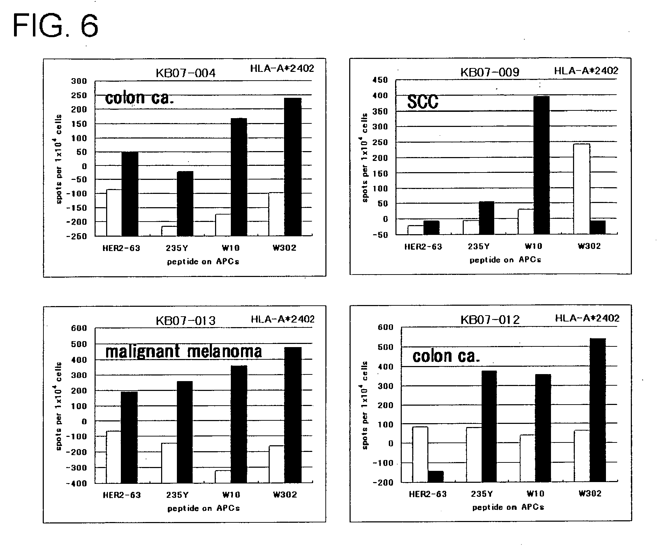

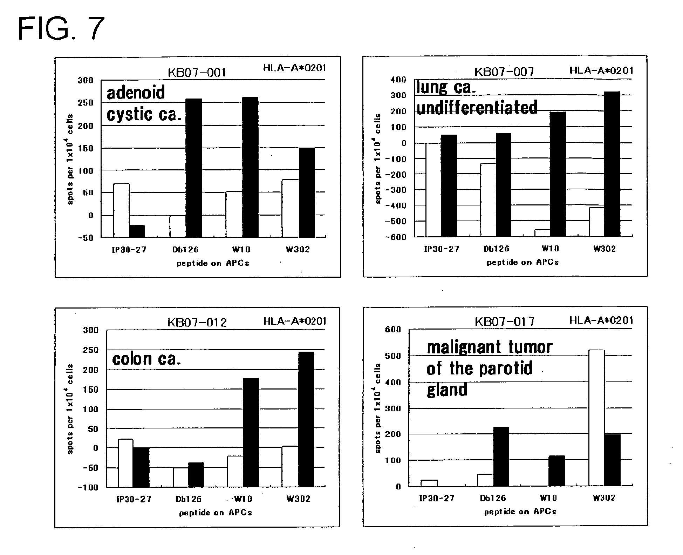

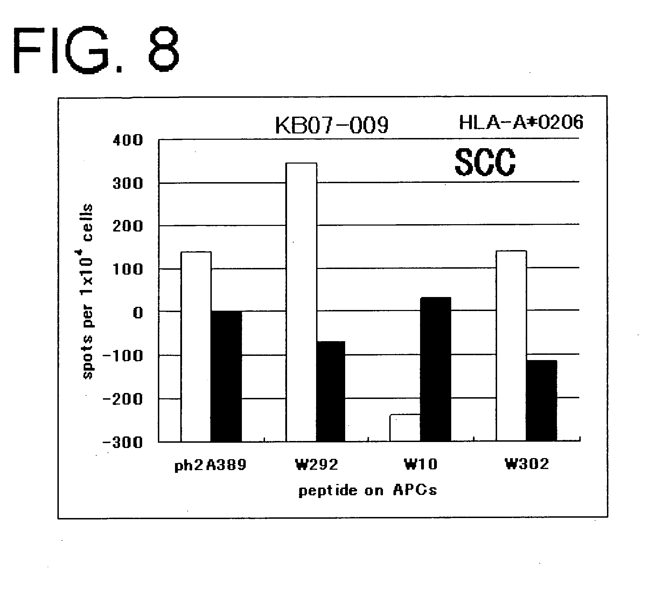

The results are shown in FIG. 6 (response of PBMC of patients having HLA-A*2402), FIG. 7 (response of patients having HLA-A*0201), and FIG. 8 (response of patients having HLA-A*0206). The abscissa denotes the peptide that was bound to antigen presenting cells (in the figures, referred to as APCs (antigen presenting cells)), and the ordinate denotes the number of INF-γ-producing cells per 1 x 10^4 cells. The response of peripheral blood mononuclear cells one week after three-time immunizations is shown with closed bars, and the response of peripheral blood mononuclear cells collected immediately before immunization is shown with open bars. The number of cells responding to the peptide was defined as the number obtained after subtracting the number of cells producing IFN-γ against APCs (FIG. 6; RA24, FIG. 7; T2, FIG. 8; RA2.6) not pulsed with peptide KB07-xxx denotes the registration No. of the respective patient. The types of tumors are given in the figures.

After immunization, the increase of cells producing IFN-γ in response to W10 peptide used for immunization was often observed among patients. Interestingly, after the immunization, a number of patients exhibited increased responses not only to the immunized W10 peptide, but also to other WT1-derived peptides.
This may be due to the induction of cytotoxic T-cells, at the local tumor sites or in the draining lymph nodes, which are specific for the WT1-derived peptides presented endogenously by the tumor cells as a consequence of an offense against tumors by the W10 peptide-reactive T cells.

### Cells used in ELISPOT assays

C1R-A24 is a cell line expressing HLA-A*2402 by introducing the gene in an HLA class I deficient cell line C1R as described above. RA24 is a cell line expressing HLA-A*2402 by transducing a mouse TAP (transporter associated with antigen processing) deficient cell line RMAS with a retroviral expression vector pLNCX2 (CLONETECH, BD Biosciences, Palo Alto, CA) bearing the cDNA (generated by Keiko Udaka et al., at Kochi Univ.). RA2.6 was generated by transfecting RMAS cells with an expression plasmid pMKITneo bearing the cDNA of HLA-A*0206 (prepared by Fumio Tsuji, Santen Pharmaceutical Co., Ltd. and Udaka, Kochi University in collaboration). T2 is a human TAP deficient cell line expressing HLA-A*0201. T2 was provided by Dr. Peter Cresswell (Yale University).

### Method to analyze the peptide reactivity of human peripheral blood mononuclear cells by ELISPOT assay

Peripheral blood mononuclear cells (PBMC) were stimulated in vitro once a week for a total of three times and then, the number of cells that produced IFN-γ in response to the antigen peptide were counted. *In vitro* stimulation was carried out by suspending PBMCs at 1 x 10^6/mL in 10 % human serum DMEM (Dulbecco's modified minimum essential medium) containing 10 U/mL of human IL-2, 5 ng/mL of human IL-7, and 100 pg/mL of human IL-12, and 200 µL of cell suspension was distributed to each well of a 96-well U-bottomed plate. HLA-binding peptide was added to a final concentration of 1 µM.

One of the WT1 peptides (W10, W302, W292), which are newly described in the present application, was used for the stimulation. Furthermore, as controls, Db126 (HLA-A*0201-binding; RMFPNAPYL) and 235Y (HLA-A*2402-binding; CYTWNQMNL), which are HLA-binding WT1-derived peptides that have already been reported, were used for the stimulation. For the second and third *in vitro* stimulation, autologous PBMCs having been cultured in DMEM containing 10 % human serum and 20 U/mL IL-2 were used as antigen presenting cells. They were treated with mitomycin C (MMC) in order to eliminate the proliferative capacuity. Peptide was then added to a final concentration of 1 µM and incubated at 37 °C for 1 hr before adding to the responder cells prepared above. On the next day of the third stimulation, 20 U/mL of IL-2 was added and incubated for 9 days before tested by ELISPOT assay.

For ELISPOT assay, a Multiscreen HTS IP plate (Millipore, Bedford, MA) was first coated with anti-IFN-γ antibody 2G1 (ENDOGEN, Pierce Biotechnology, Rockford IL), and 5, 000 cells of the *in vitro* stimulated PBMCs as described above or a twofold or fourfold dilution thereof (2,500 or 1,250 PBMCs, respectively) were placed in each well together with the antigen presenting cells which have been loaded with a peptide to be tested and then treated with MMC. Cells were incubated at 37 °C overnight. As antigen presenting cells expressing the respective HLA molecules, RA24 (HLA-A*2402), T2 (HLA-A*0201), and RA2. 6 (HLA-A*0206) were used. On the next day, the cells were removed and biotin-labeled another anti-IFN-γ antibody B133.5 (ENDOGEN) was added to bind to the plate-bopund IFN-γ. HRPO (horseradish peroxidase)-labeled streptavidin (Mabtech, Cincinnati OH) was then added to bind thereto, and the number of IFN-γ-producing cells were counted after the enzyme reaction. TMB (tetramethylbenzidine) was used as a substrate.

As control HLA-binding peptides that were used to load antigen presenting cells, those below were used.
HLA-A*0201 binding peptides: IP30-27; LLDVPTAAV and Db126; RMFPNAPYL, HLA-A*2402 binding peptides: HER2-63; TYLPTNASL and 235Y; CYTWNQMNL, HLA-A*0206 binding peptides: ph2A389; SLLPAIVEL and W292; GVFRGIQDV (W292 is a WT1-derived peptide, thus it was used also as test peptide as well as a control peptide for other WT1-derived peptides). Here, in the wells where APCs were loaded with IP30-27, HER2-63, and ph2A389 as a control peptide, responder cells had not been stimulated with any peptide.

### <Measurement of killing activity of PBMCs against peptide-loaded APCs by ⁵¹Cr (chromium 51) assay>

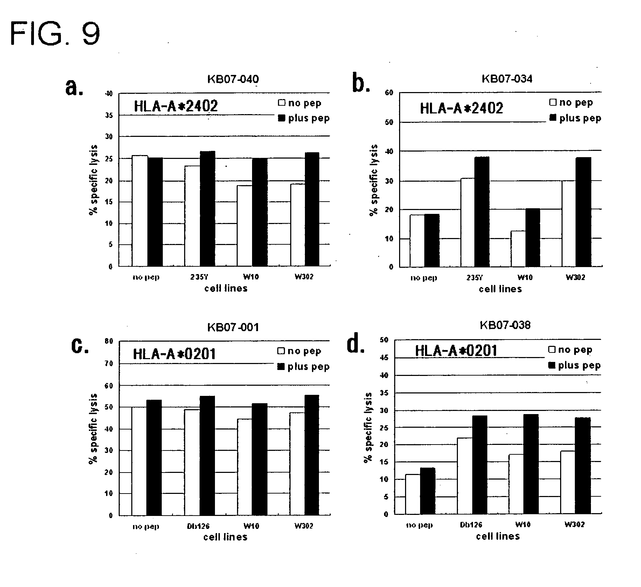

As a next experiment, PBMCs from patients with malignant tumors were stimulated with peptide *in vitro* and their cytolytic activity was examined against peptide-loaded tatrget cells by ⁵¹Cr release assay described later. As shown in FIG. 9, PBMCs from the patients bearing malignant tumors contain peptide-reactive cytolytic cells.

These cytolytic cells include those reactive to HLA-A*2402 binding WT1 peptides, 235Y, W10, and W302 as shown in Fig. 9a and b. Furthermore, PBMCs from patients with malignant tumors contain cytolytic cells that recognize HLA-A*0201 binding WT1 peptides, Db126, W10, and W302. Also in the cytolysis assay a number of patients were found to have an elevated reactivity not only to W10 peptide that was used for *in vitro* stimulation, but also against other WT1-derived peptides.

### Method of assessment of cytolytic activity by ⁵¹Cr release assay

Target cells were labeled with ⁵¹Cr and loaded with HLA-binding peptide of interest. 5,000 or 10,000 cells/well of target cells were placed in 96-well U-bottom plate, and then, PBMCs having been stimulated twice *in vitro* were added thereto as a responder. Cells were incubated at 37 °C for 5 hours and ⁵¹Cr released from the damaged cells during the incubation was measured, and % specific lysis was calculated. As target cells C1R-A24 or RA24 cells were used as a target when reactivity to HLA-A*2402 binding peptides were examined, and T2 cells were used as a target and incubation was carried out for 2 hours after addition of responder cells. Percent specific lysis was calculated as follows. % specific lysis = (test release-spontaneous release)/(total release-spontaneous release) x 100.

### <Treatment for patients>

Among 18 patients, 3 people are illustrated in the style of case reports. After confirming safety by the above-mentioned safety test, all patients were injected intradermally once a week in principle (there were occasions in which it was missed by 1 to 2 days), at 4 locations (2 locations close to axillary lymph nodes, 2 locations in groin) with 100 µL each of the mixture of 300 µL (3 mg equivalent) of 10 mg/mL W10 peptide in 5 % glucose and 100 µL (5 x 10^8 cells) of 5 x 10^9 cells/mL of whole-cell pertussis vaccine in saline. The mixture was prepared immediately prior to administration. When W10 peptide was administered without petussis vaccine in the safety test, W10 peptide solution was divided into 4 equal portions and injected intradermally at 4 locations in the same manner. A method to measure the diameter of a tumor and a method to analyze peptide-specific T-cells using an HLA-A24 tetramer by flow cytometry are described below.

### Case report 1

KB07-006 66 years old female, Adenoid cystic carcinoma, stage 4C lung metastasis

The primary tumor was positive for WT1 expression as determined by immunohistochemical staining. HLA-A*2402 positive.

### Clinical course before starting the W10 peptide immunotherapy

The primary tumor on the oral floor was subjected to a combined therapy of an arterial infusion of anticancer drug and radiation. In response to the therapy, central necrosis was observed. However, residual tumor existed and in addition, a metastatic tumor appeared in the lung. IFN-γ, IL-2, LAK therapy was carried out, but there was no change, and the tumor continued to grow. Therefore, previous therapy was terminated and one month later, immunotherapy was started using a known WT1 tumor peptide, 235Y (amino acid sequence: CYTWNQMNL). Three mg of 235Y peptide was emulsified with Montanide (component being Freund's incomplete adjuvant) in order to impart sustained-release properties, and injected intradermanlly once a week. An apparent reduction of the primary tumor was observed by MRI on the 4th week after starting the immunization.

Immunization was continued once a week. Then, the primary tumor has disappeared and at present, three and a half years after the therapy, there is no recurrence. The single metastasis in the lung did not change in size while 235Y peptide immunization was continued for 10 months, and was determined to be stable disease (SD) by the international RECIST criteria for evaluating an anticancer drug, which will be described later. At 10th month after starting 235Y peptide immunotherapy the immunotherapy was suspended on a request of the patient. After the suspension, the lung metastasis as well as metastasis to other parts of the body, such as in the iliac bone started to develop. At one year and seven months after the suspension the number of lung metastatic tumors increased to a total of 18, and the total sum of the longest diameters of 10 target tumors, which sizes could be measured precisely, reached 16.3 cm.

The international RECIST criteria referred to here is an evaluation standard described in Theresse, P. et al., J Natl Cancer Inst. 2000, 92, 205-216. When there is confirmation by helical CT or MRI that all target lesions have disappeared this is defined as CR (complete response), when the sum of the longest diameters of the target lesions decreases by at least 30 % this is defined as PR (partial response), when the sum of the longest diameters of the target lesions increases by at least 20 % this is defined as PD (progressive disease), and when the change of tumr size is between PR or PD this is defined as SD (stable disease). However, regardless of the presence or absence of a change in the target lesions, when even one new lesion appears this is defined as PD.

Criteria for determining an adverse event is in accordance with CTCAE (Common Terminology Criteria for Adverse Events v3. 0) by JCOG/JSCO-Oct. 27, 2004; when the grade was 2/3 the test therapy was suspended, and when the grade was 4 or greater, the test therapy was terminated regardless of causal relationship to the test therapy.

### Clinical course after starting W10 peptide immunotherapy

At this point, immunotherapy using W10 (ALLPAVPSL) peptide was started. During the first 3 weeks, 1 mg of W10 peptide was administered on its own once a week, subsequently immunization was carried out three times by adding to 1 mg of W10 peptide 5 x 10^8 cells of whole-cell pertussis vaccine (hereinafter, described as 'Wc', the amount administered being the same) as an adjuvant, and the safety was monitored.

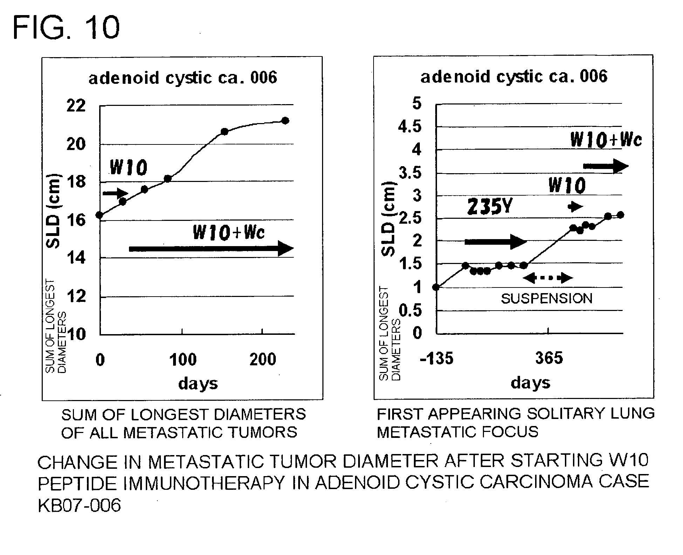

Following this, an administration method was fixed with 3 mg of W10 peptide + Wc, and immunization was continued once a week. From the results, as shown in FIG. 10, the sum of the longest diameters of the all target tumors, and the longest diameter of the first appearing lung metastatic focus gradually slowed in speed of growth so that there became hardly any change. There has been no appearance of a new metastatic lesion up to the present, 13 months after starting W10 peptide administration. At present, although the growth is slower, compared with the value prior to the therapy, the total sum of the longest diameters of 10 metastatic lesions selected as target tumors was determined to be SD up to 32 weeks, but by CT on the 37th week there was an increase of at least 20 % compared with that prior to the therapy. Therefore, it was determined to be progressive disease (PD). In FIG. 10, in accordance with the international RECIST criteria, changes over time of the total sum of the longest diameters of 10 target tumors (left diagram) and the longest diameter of the first appearing single metastatic tumor (right diagram) are shown. Arrows inserted into the diagrams denote the duration of the respective immunotherapies. For the safety test, it was necessary to carry out stepwise an increase in the amount of peptide and the combined use of Wc.

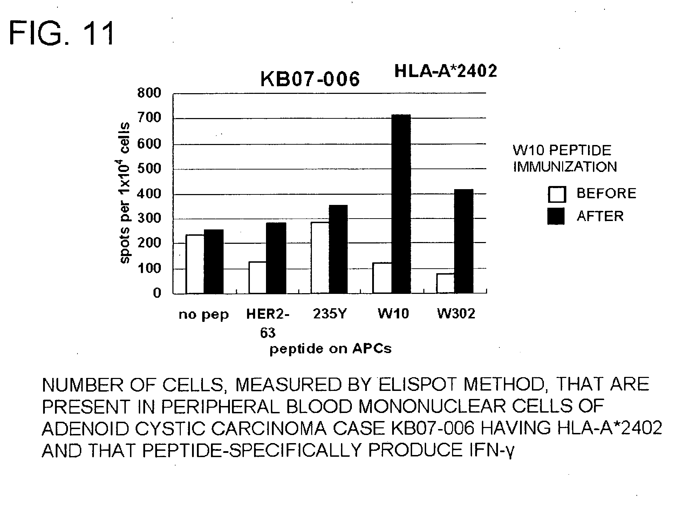

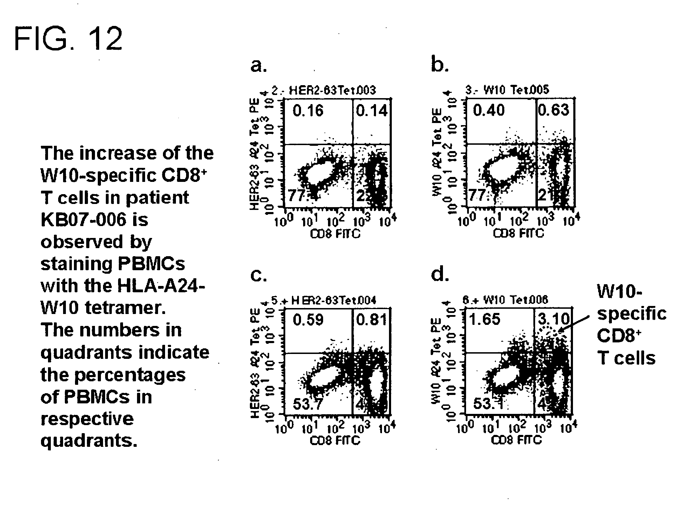

Reactivity toward W10 of PBMCs from this patient was investigated by an ELISPOT assay; as shown in FIG. 11, the number of W10-reactive IFN-γ-producing T-cells was found to have increased after administrating 1 mg of W10 peptide for 3 weeks. Moreover, when an HLA-A24-W10 tetramer molecule was fluorescence-labeled by i.e. PE (phycoerythrin)-labeled SA (streptavidin) as was done here and used to stain the T cells specific for HLA-A24-W10 complex to identify antigen specific T cells, W10-specific CD8 positive T cells (cytotoxic T cells) were found to have increased in the culture of PBMCs having been incubated in the presence of W10 peptide (Fig. 12).

From these observations, it was found that growth of tumors expressing WT1 was suppressed as a result of W10 peptide immunotherapy. On the other hand, no adverse event due to peptide immunotherapy was observed, except for an erythema and mild swelling at the site of immunization which was most obvious after 2 days. These results suggest that as a result of the immunotherapy employing the W10 peptide, W10-reactive CD8-positive cytotoxic T cells increased, and growth of the tumor was suppressed.

### Case report 2

KB07-005 51 years old male, Prostate cancer (hormone therapy-resistant undifferentiated cancer)

The tumor was positive for WT1 expression as determined by immunohistochemical staining. HLA-A*2402 positive.

### Clinical course before starting W10 peptide immunotherapy

In October 2005, after radical prostatectomy, there has been no local recurrence, but in October 2006 a metastatic tumor appeared in the right femur. Radiation therapy (42 Gy) was carried out for the purpose of pain relief. From December 2006, WT1 peptide (235Y) immunotherapy was started. There was no effect, and the tumor kept growing. In March 2007 radiation therapy (36 Gy) was added for pain relief.

### Clinical course after starting W10 peptide immunotherapy

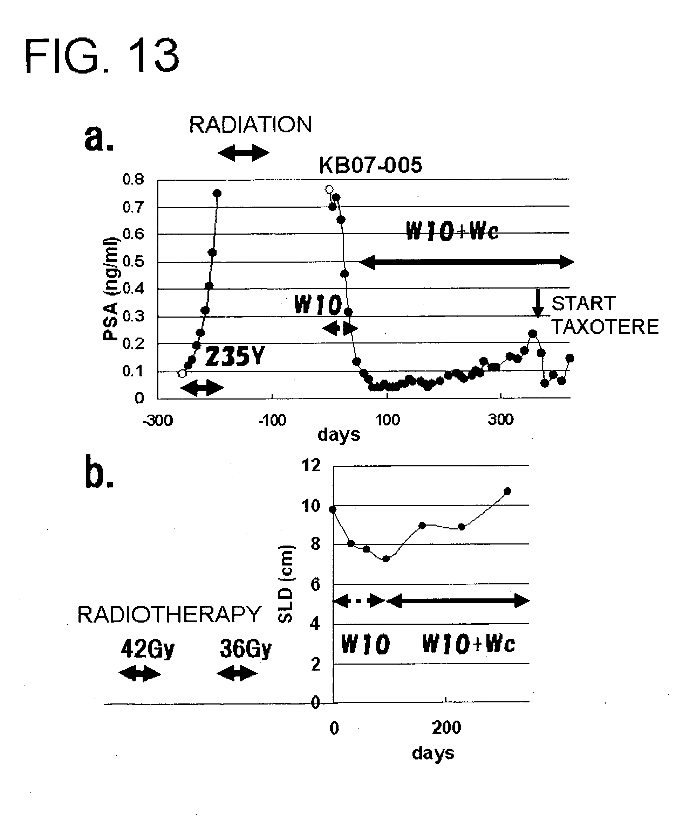

A new WT1 peptide (W10) immunotherapy was started from October 2007 (the amount being gradually increased after three administrations while checking safety; after W10 peptide on its own was gradually increased from 1 mg to 3 mg, it was gradually increased from W10 peptide 1 mg + Wc to W10 peptide 3 mg + Wc). After the gradual increase the dose was fixed to 3 mg of W10 peptide + Wc and immunotherapy was continued. FIG. 13 a. shows a change in a prostate tumor-specific marker, prostate-specific antigen (PSA). The day when 1 mg of W10 peptide on its own was first administered was presented as day 0. Arrows with dotted line indicates the period when 3mg of W10 without Wc was injected for safety test (W10). The period where Wc was further added to W10 is also indicated with arrows with solid line (W10+Wc). After starting the immunotherapy, PSA rapidly decreased and was stabilized at around 0.04 (FIG. 13a). FIG. 13 b. shows the sum of the longest diameters of metastatic tumors in the right femur (sum of the longest diameters; SLD). This patient had a hormone-therapy resistant, undifferentiated cancer and it spread in an osteolytic fashion. Therefore, osteolytic lesions were measured as the size of tumors. The growth of metastatic tumor in the bone almost stopped (FIG. 13 b) and the pain was also alleviated. Around May 2008, PSA gradually started to increase again, and pain started in the right femur. From the end of July 2008, a taxotere + Predonin therapy was started. W10 + Wc immunotherapy was continued along with chemotherapy. From the time the immunotherapy was started using W10 peptide, no new distant metastasis was observed throughout the period.

### Case report 3

### KB07-003 58 years old male, Prostate cancer (PSA positive, hormone therapy-resistant)

The tumor was positive for WT1 expression as determined by immunohistochemical staining. HLA-A*0201 and HLA-A*2402 positive.

### Clinical course before starting W10 peptide immunotherapy

In August 2003, radical prostatectomy was carried out. After the operation, PSA increased and bone metastasis appeared, thus, hormone therapy was carried out. The therapy was effective for a while, but the tumor became gradually resistant to the therapy and in March 2005 and thereafter, PSAcontinued to rise. In May to June 2007 the PSA was normalized by radiotherapy and hormone therapy. From the end of August, W10 peptide immunotherapy was started.

### Clinical course after starting W10 peptide immunotherapy

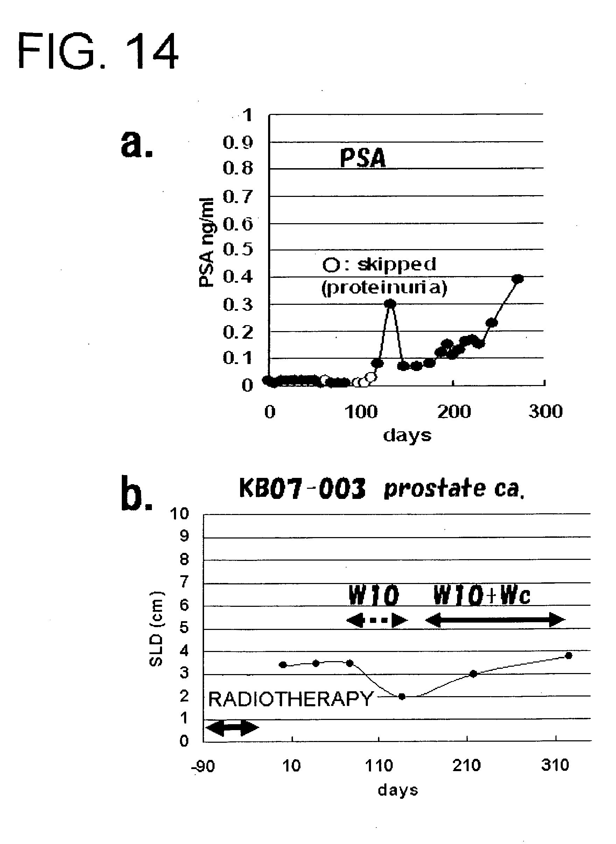

Immunotherapy was started with 3 mg W10 peptide while monitoring safety, then dose escalation was put forward with W10 peptide 1 mg + Wc, followed by 3 mg of W10 peptide + Wc. Eventually, the dose was fixed at this level and weekly immunization was continued. FIG. 14 a. shows change in PSA. The day the immunization was started with 3 mg of W10 without Wc was set as day 0. Data points denoted by open circles indicate the occasion when W10 immunotherapy was suspended due to a Grade 2 increase of Cre (creatinine: creatinine) most likely caused by diabetic nephropathy. For about half a year, PSA was stable at less than 0.1 (FIG. 14 a). However, when the immunotherapy was suspended three times in succession due to a Grade 2 increase of Cre, PSA rapidly increased. When the immunotherapy was resumed, PSA decreased. After this incident, PSA started to show gradual increase. FIG. 14 b. shows the sum of the longest diameters (SLD) of metastatic tumors in the bone. The size of a pubic bone metastatic tumor has been maintained within SD for 37 weeks on the RECIST standard so far (FIG. 14 b). Since the W10 peptide immunotherapy was started, no new metastatic tumors have been found. The period of radiation therapy for the purpose of pain relief is denoted by arrows

### Method for measuring tumor diameters

Based on the above-mentioned international RECIST criteria, which is used for evaluation of anticancer drugs, all measurable tumors were selected as target tumors, and change in the SLD of the tumors over the course of the therapy was monitored. In addition, number of all the tumors was counted, regardless of the size of a tumor, every time diagnostic imaging was carried out in order to lookout for new lesions. Diagnostic imaging was carried out in principle once a month by helical CT or MRI. If even one new metastasis appeared, regardless of the change in the primary lesion, it was judged as PD.

Analytical method to identify peptide-specific T-cells using a HLA-A24 tetramer by flow cytometry

HLA-A24 tetramer was prepared by modifying a method by Altman et al. (Altman, JD, Moss, PA., Goulder, PJ, Barouch, DH, McHeyzer-Williams, MG, Bell, JI, McMichael, AJ, Davis, MM. Phenotypic analysis of antigen-specific T lymphocytes. Science 1998 274, 94-96.)

HLA-A24 tetramer was prepared by expressing an HLA-A*2402 molecule in *E. coli* and W10 peptide was bound thereto. Furthermore, the C-terminus of the complex was modified by a site-specific biotinyltion. The product was then complexed with streptavidin (SA), giving rise to a molecular assembly consisting of 4 molecules of W10-bound HLA-A*2402 molecules and 1 SA.
Since the binding affinity of T cell receptor (TCR) to an antigen is very low, a single HLA-A24-W10 complex is not expected to bind stably, but an HLA tetramer consisting of 4 moledules of it binds polyvalently to TCRs, thus forming a stable complex with T cells.
Here in this assay, T cells specific for a HLA-A24-W10 complex was fluorescently labeled with RE (phycoerythrin) conjugated SA and antigen specific T cells were identified by flow cytometry.
cDNAs of HLA-A*2402, which is an H-chain constituting an HLA-A*2402 molecule, and human β2m (β2-microglobulin) which is a light chain, were inserted into a pET-21d+ vector (Novagen, Merck) under T7 promoter, respectively. The resultant vector was introdyced into *E. coli* BL21 (DE3) pLysS, and expression of the gene was induced using IPTG. The recombinant protein produced as an inclusion body was dissolved using 8 M Urea in the presence of protease inhibitors and mixed with β2m, that had been similarly expressed in *E. coli,* and W10 peptide at a molar ratio of 1:2:10. The mixed molecules were refolded by a stepwise dilution method. Correctly assembled HLA-A*2402-W10 monomer was purified by means of DEAE-Sepharose chromatography, and 1 molecule of biotin was covalently bound to the C terminal portion of the HLA-A*2402 chain using the site-specific biotinylation enzyme, BirA

This HLA-A*2402-W10 monomer was associated with PE-labeled SA at a molecular ratio of 4:1, thus preparing HLA tetramer. A control HLA-A*2402 tetramer was prepared using HER2-63 peptide, which is HLA-A*2402-binding, but not related to WT1, in place of W10. T cells were incubated with HLA-A*2402-W10 or HLA-A*2402-HER2-63 on ice for 1 hour and then analyzed by flow cytometry.

### <Evaluation of therapeutic effect>

Clinical responses were evaluated according to the aforementioned international RECIST criteria for 18 patients who had been treated by immunotherapy using W10+Wc for at least 3 months and one week. Eight cased out of 18 patients were judged as SD, 10 cased were PD. The tumor control rate, as defined as the percentage of patients exhibiting the clinical response of SD or better, was 44 % (Table 4). In reports on peptide immunotherapy so far, the tumor control rate was limited to a few % up to ten or so % worldwide (Reference Documents 1 to 4) . Compared with these already reported cases, this is a good reaction.

**[Table 4]**

| Case No. | Name of disease | Duration of tumor of tumor control (weeks) | of W10 peptide therapy | Progress | RECIST determin ation 3 months after starting W10 peptide therapy |
|---|---|---|---|---|---|
| 1 | Adenoid cystic carcinoma | 33 | 44 | SD by CT even after even after 33 weeks | SD |
| 2 | Extramammary Paget's disease | 38 | 52 | | SD |
| 3 | Prostate cancer | 37 | 48 | If drug suspended PSA ↑ | SD |
| 5 | Prostate cancer | 30 | 52 | SD by MRI after 30 weeks | SD |
| 6 | Adenoid cystic carcinoma | 32 | 52 | D after 32 weeks, PD at 37^{th} week | SD>PD |
| 7 | Large cell lung cancer | 10 | 10 | | PD |
| 11 | desmoplastic s.r.t | 4 | 14 | | PD |
| 12 | Colon cancer | 0 | 9 | Tumor marker sustained increase | PD |
| 13 | Malignant melanoma | 0 | 13 | Primary focus unchanged, new lesions | PD |
| 17 | Submandibular gland cancer | 0 | 6 | Lung, bone, liver metastasis | PD |
| 18 | glioblastoma | Impossibl e to determine (**) | 15 | prevention of postoperative relapse | PD |
| 19 | gliomatosis cerebri | 6 | 42 | Control of growth rate, continuous treatment | PD |
| 21 | Prostate cancer | 17 | 30 | Tumor marker relapse case | SD |
| 22 | Prostate cancer | 0 | 10 | Tumor marker reduction → increase | PD |
| 24 | Sacral chordoma | 19 | 24 | | SD |
| 35 | Lung cancer (SCC) | 12 | 16 | 5/20 pleural fluid ↓, symptoms improved | SD |
| 38 | Malignant melanoma | 0 | 12 | | PD |
| 40 | Bone spindle lung metastasis | cs, 0 | 21 | | PD |

| | | | | | |
|---|---|---|---|---|---|
| * The tumor control rate, as defined as the percentage of patients exhibiting the clinical response of SD or better, was 44 % (4 cases among the 18 cases). ** In case 18, there was no postoperative relapse. Thus, evaluation by the RECIST criteria was not possible. | | | | | |

### Reference Documents 1 to 4

1. Mosolits, S., Ullenhag, G., Mellstedt, H. Therapeutic vaccination in patients with gastrointestinal malignancies. A review of Immunological and clinical results. Ann Oncol 16, 847-862, 2005
2. Nagorsen, D. and E. Thiel. Clinical and immunologic responses to active specific cancer vaccines in human colorectal cancer. Clin Cancer Res 12, 3064-3069, 2006 3. Rosenberg, S.A., Yang, J.C., Schwartzentruber, D.J., Hwu, P., Marincola, F.M., Topalian, S.L., Restifo, N.P., Dudley, M.E., Schwarz, S.L., Spiess, P.J., Wunderlich, J.R., Parkhurst, M.R., Kawakami, Y., Seipp, C.A., Einhorn, J.H., White, D.E. Immunologic and therapeutic evaluation of a synthetic peptide vaccine for the treatment of patients with metastatic melanoma. Nat Med 10, 909-915, 2004 4. Slingluff, C. L., Petroni, G.R., Yamshchikov, G.V., Barnd, D.L., Eastham, S., Galavotti, H., Patterson, J.W., Deacon, D.H., Hibbitts, S., Teates, D., Neese, P.Y., Grosh, W.W., Chianese-Bullock, K.A., Woodson, E.M., Wiernasz, C.J., Merrill, P., Gibson, J., Ross, M., Engelhard, V.H. Clinical and immunologic results of a randomized phase II trial of vaccination using four melanoma peptides either administered in granulocyte-macrophage colony-stimulating factor in adjuvant or pulsed on dendritic cells. J Clin Oncol 21, 4016-4026, 2003

The present invention is explained above by reference to Examples. These Examples are only illustrated as examples, and a person skilled in the art will understand that various modification examples are possible, and such modification examples are included in the scope of the present invention.

This application claims priority based on Japanese Application No. 2007-301000 made on 20th November 2007, the content of which is incorporated hereinto by reference.

## Claims

1. A method for inducing cytotoxic T-cells, the method comprising:
binding to an HLA molecule on the surface of a cell that is a target of a cytotoxic T-cell a peptide comprising one or more types of amino acid sequence selected from the group consisting of SEQ ID NOS: 1, 2, and 3 and composed of not less than 8 and not more than 11 amino acid residues, or a peptide derived from a precursor thereof.

2. The method as set forth in Claim 1,
wherein the peptide or the peptide derived from a precursor thereof binds to a human HLA-A*2402 molecule on the surface of the target cell.

3. The method as set forth in Claim 1,
wherein the peptide or the peptide derived from a precursor thereof binds to a HLA-A*0201 molecule on the surface of the target cell.

4. The method as set forth in Claim 1,
wherein the peptide or the peptide derived from a precursor thereof binds to a HLA-A*0206 molecule on the surface of the target cell.

5. A cytotoxic T-cell inducer comprising at least one of a peptide and a precursor thereof, the peptide comprising one or more types of amino acid sequence selected from the group consisting of SEQ ID NOS: 1, 2, and 3, composed of not less than 8 and not more than 11 amino acid residues, and binding to an HLA molecule on the surface of a cell that is a target of a cytotoxic T-cell.

6. A pharmaceutical composition for the treatment of a malignant tumor, the composition comprising at least one of a peptide and a precursor thereof, the peptide comprising one or more types of amino acid sequence selected from the group consisting of SEQ ID NOS: 1, 2, and 3, composed of not less than 8 and not more than 11 amino acid residues, and binding to an HLA molecule on the surface of a specific malignant tumor cell.

7. A vaccine used for the prevention or treatment of a malignant tumor, the vaccine comprising at least one of a peptide and a precursor thereof, the peptide comprising one or more types of amino acid sequence selected from the group consisting of SEQ ID NOS: 1, 2, and 3, composed of not less than 8 and not more than 11 amino acid residues, and binding to an HLA molecule on the surface of a specific malignant tumor cell.
